# EUROPEAN PATENT SPECIFICATION

(11) **EP 0 279 161 B1**
(45) Date of publication and mention of the grant of the patent: **05.01.1994**
(21) Application number: 88100081.4
(22) Date of filing: 05.01.1988
(51) Int. Cl.: C07D 233/64, A61K 31/415

(54) **A process for the preparation of cimetidine**
Verfahren zur Herstellung von Cimetidin
Procédé de préparation de cimétidine

(30) Priority: 23.01.1987 YU 91/87
(43) Date of publication of application: 24.08.1988
(73) Proprietor: LEK, tovarna farmacevtskih in kemicnih izdelkov, n.sol.o., SI-61107 Ljubljana (SI)
(72) Inventor: Zizek, Teofil, M. Sc., YU-61000 Ljubljana (YU)
(74) Representative: Müller-Boré & Partner Patentanwälte

(56) References cited:
- WO-A-80/01694

## Description

### Technical Field of the Invention

### (IPC C 07 D 233/64)

The present invention relates to a new process for the preparation of crystalline cimetidine, i.e. N-cyano-N'-methyl-N"-( 2/(5-methyl-1 H-imidazole-4-yl)methylthio/ethyl;j -guanidine of the formula 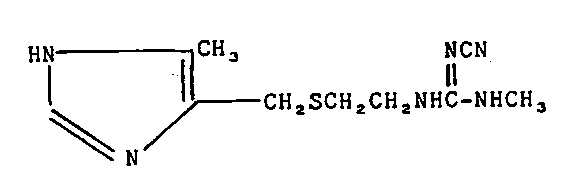 Cimetidine is an exceptionally efficient antagonist of histamine on H₂ receptors. It has a hindering effect upon the secretion of gastric acid and for this reason it is used in the therapy of the ulcer disease.

### Technical Problem

There exists a constant need for a technologically advantageous new process for the preparation of crystalline cimetidine, which would afford the desired compound in an excellent yield, with high purity and in a form that can be easily isolated from the reaction mixture.

### Prior Art

Cimetidine (N-cyano-N'-methyl-N"- 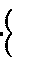 2/(5-methyl-1H-imidazole-4-yl)methylthio/ethyl 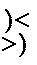 guanidine) is a known compound, which was disclosed for the first time in GB patent 1,338,169. In GB patent of addition 1,397,436 to the former GB patent, there are also disclosed processes for the preparation of cimetidine. These processes, which are schematically shown on page 3, are based on the reaction of 4-halomethyl-, 4-hydroxymethyl- or 4-methoxymethyl-5-methylimidazole and its hydrohalogenide, respectively, with cysteamine hydrochloride (in this scheme X in formula I stands for a halo, a hydroxy or a methoxy group).

The reaction results in the formation of the intermediate compound 4-/(2-aminoethyl)-thiomethyl/-5-methyl-imidazole and its dihydrohalogenide, respectively, which is reacted with methyl isothiocyanate to form N-methyl-N'- 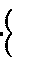 2/(4-imidazolyl-5-methyl)methylthio/ethyl 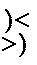 thiourea, which is then reacted with lead cyanamide to yield cimetidine.

Cimetidine can also be obtained by reacting 4-/(2-aminoethyl)-thiomethyl/-5-methyl-imidazole with N-cyano-N',S-dimethylisothiourea or with dimethylcyanodithioimido carbonate to form N-cyano-N'-{ 2-/(5-methylimidazole-4-yl)methylthiolethyl) -S-methylisothiourea, which is reacted with methylamine to yield the desired compound. Among these processes the last one provides the best results, the overall yield of the reaction being up to 60%.

There are described several more processes for the preparation of cimetidine in the patent literature. In DE Offenlegungsschrift 28 55 836 there is described a process which is based on the reaction of 4-methyl-5-mercaptomethyl imidazole with 1-(N-methyl-N'-cyanocarboxamidino)-aziridine and on the reaction of 4-methyl-5-mercaptomethyl imidazole (in the form of sodium mercaptide) with N-(2-bromoethyl)-N'-methyl-N"-cyanoguanidine,respectively, in an ethanolic solution.

The starting 4-methyl-5-mercaptomethyl imidazole is prepared in a known manner, which is described in the literature for the synthesis of thiols by converting 4-ethoxy-dithioformyl-2,3-diketo-butane to 4-methyl-5-(ethoxy-dithioformyl)imidazole, 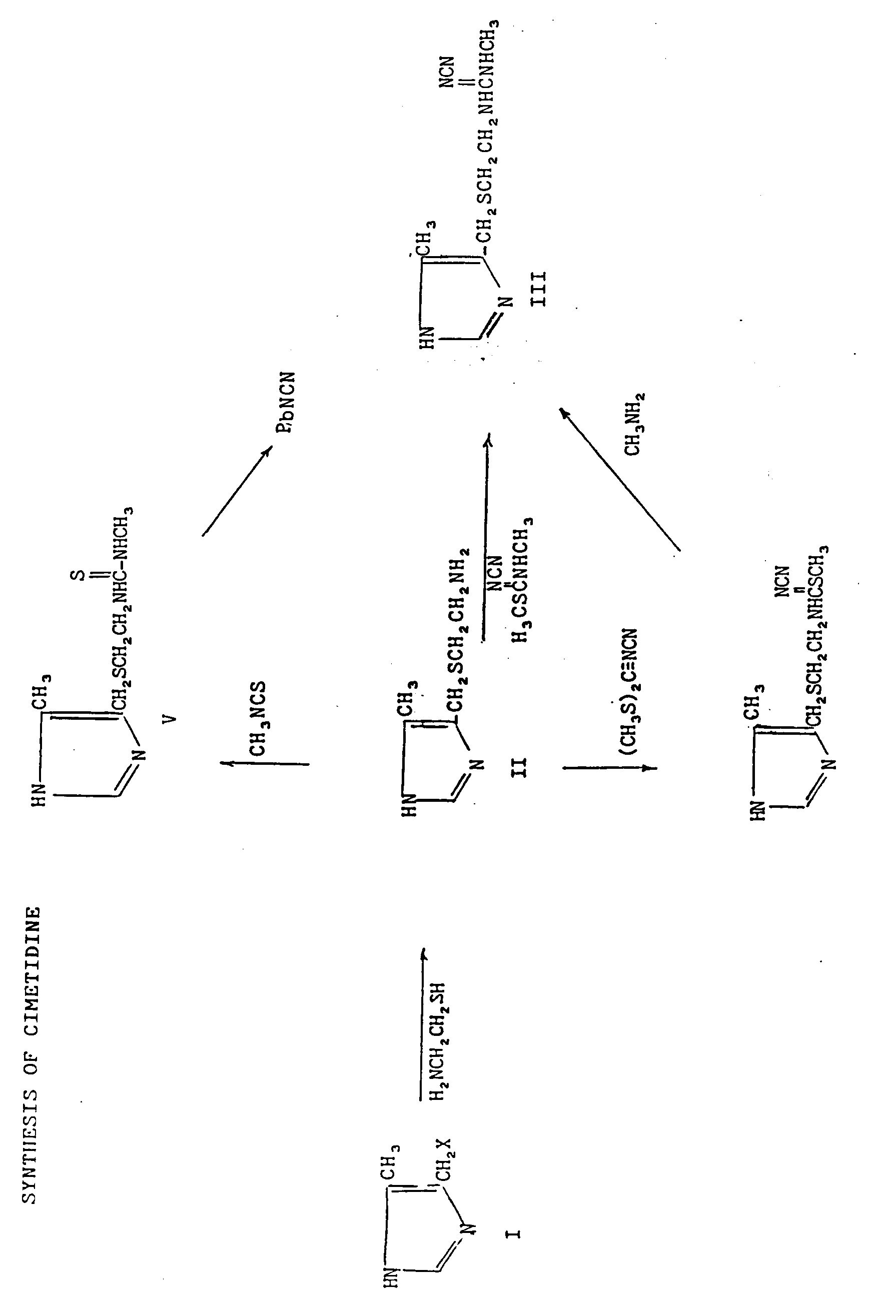 which is then hydrolysed with HCI at the reflux temperature of the reaction mixture under a nitrogen atmosphere to give 4-methyl-5-mercaptomethyl imidazole hydrochloride in a yield of 70 % (Examples 1-3 of the above DE Offenlegungsschrift). In Example 6 of the same application a variant of this process is described, wherein 4-methyl-5-chloromethyl imidazole is reacted with potassium ethylxanthate in an ethanolic solution at the reflux temperature of the reaction mixture to form 4-methyl-5-(ethoxy-dithioformyl)-imidazole, which is then hydrolysed as described above to yield 4-methyl-5-mercaptomethyl imidazole hydrochloride.

A drawback of said process is the preparation of the starting 1-(N-methyl-N'-cyanocarboxamidino)-aziridine using the hazardous, very toxic and even carcinogenic aziridine (ethyleneimine).

In the second variant the results are unsatisfactory due to the instability of N-(2-bromoethyl)-N'-methyl-N"-cyanoguanidine.

A further process, described in the CA Reissue 1,209,153 of the patent 1,121,363, is based on the reaction of 5-methyl-4-thiomethyl imidazole (in the form of its hydrochloride salt) with N-cyano-N'-methyl-N"-(2-chloroethyl)guanidine. The process provides the desired compound in a high yield, avoiding at the same time the use of the disadvantageous reactants, which are used in the DE Offenlegungsschrift 28 55 836.

A drawback of this process as well as of the process of DE-OS 28 55 836 is the handling of 5-methyl-4-thiomethyl imidazole because of the known properties of thiol compounds, which have very unpleasant odour and are environmental polluants. Also some direct ways for the synthesis of the intermediate thiol as described in Example 4 of the above Canadian patent involve the use of thiol starting compounds such as KSH, which require additional precautionary measures in order to prevent emissions into the environment.

### Description of the Solution to the Technical Problem with Examples

The object of the invention is the preparation of crystalline cimetidine in a new and simple manner, wherein the working with toxic thiols is avoided, by the reaction of O-ethyl-S-(4-methyl-imidazolyl-5-methyl)-dithiocarbonate (in the form of its hydrobromide salt) with N-cyano-N'-methyl-N"-(2-chloroethyl)guanidine in a excellent yield, with high purity and a simple isolation from the reaction mixture. This object is achieved by reacting the above two compounds at a temperature between room temperature and the reflux temperature of the reaction mixture in an aqueous methylamine solution, which maintains the basicity of the medium.

O-ethyl-S-(4-methyl-imidazolyl-5-methyl) dithiocarbonate (as hydrobromide) of the formula 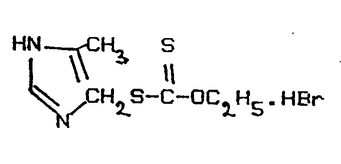 was prepared in accordance with the process described in US patent 4,720,552. 4(5)-methyl-5(4)-methyl-5-(4)-bromomethyl imidazole hydrobromide, which is a known compound and described in the literature (H.G. Lennartz, W. Schunack, Arch. Pharm. (Weinheim) 310, 1019-1022, 1977; P. Kairisalo, E. Honkanen, Arch. Pharm. (Weinheim) 316, 688-690, 1983; R. Tozo et al, Gazz. Chim. Ital. 109, 529-533, 1979) was reacted with potassium O-ethyl dithiocarbonate in an aqueous medium at room temperature to give, in a nearly quantitative yield, the intermediate O-ethyl-S-(4-methyl-imidazolyl-5-methyl)dithiocarbonate, which was separated by centrifugation, washed with some water and dried.

According to the above US patent the intermediate compound is, without isolation, immediately hydrolysed in the presence of a diluted aqueous solution of HBr or HCI to 4(5)-methyl-5(4)-thiomethyl imidazole in the form of its hydrobromide or hydrochloride salt, respectively. The obtained compound is then used as the key compound in the process for the preparation of cimetidine as described in the above CA patent and DE-OS.

The present inventor has found out that in contrast to the known process, it is not necessary to hydrolyse the intermediate O-ethyl-S-(4-methyl-imidazolyl-5-methyl) dithiocarbonate in acidic medium to 4-methyl-5-thiomethyl imidazole. Instead, it can be reacted with N-cyano-N'-methyl-N"-(2-chloroethyl) guanidine in the presence of an aqueous methylamine solution at a temperature between room temperature and the reflux temperature of the reaction mixture to directly yield cimetidine. In the process the aqueous methylamine solution acts as a solvent and as an agent for maintaining the basicity of the medium. A further advantage is a simple isolation of crystalline cimetide in a pure form, which renders any further purification by recrystallisation superfluous.

The second reactant, i.e. N-cyano-N'-methyl-N"-(2-chloroethyl)guanidine is advantageously prepared in the manner described in the YU patent application P-2151/83 and in the article by J. Zmitek et al., Org. Prep. Proced. lnt. Vol. 17, No. 4-5 (1985), 256-261.

N-cyano-N'-methyl-N"-(2-chloroethyl)guanidine can be prepared using dimethyl-(N-cyano-imido)-carbonate of the formula

### (CH₃O)₂C=NCN

as the starting compound. Dimethyl-(N-cyano-imido)-carbonate is a known compound. The preparation thereof is described e.g. in J. Org. Chem. 1974, 39, 1522. According to the process described in YU patent application P-2151/83, dimethyl-(N-cyanoimido) carbonate is reacted with 2-chloroethylamine hydrochloride to N-cyano-N-(2-chloroethyl)-O-methyl-isourea, which is then reacted with methylamine to yield N-cyano-N'- methyl-N"-(2-chloroethyl)guanidine.

In order to make the reaction proceed at all, it is important in which order the reactants are added since in situ there is formed 4(5)-methyl-5(4)-thiomethyl-imidazole, which immediately reacts with N-cyano-N'- methyl-N"-(2-chloroethyl) guanidine to yield cimetidine. Thereat no emission of the thiol compound to the environment can be detected. Thus the process of the present invention excels in that no malodorous thiols are handled in any reaction step, which stands in contrast to the above prior art processes.

The invention is illustrated by the following Example.

### Example

N-cyano-N'-methyl-N"- 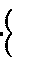 2/(5-methyl-1 H-imidazole-4-yl)methyl-thio/ethyl 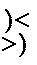 guanidine

N-cyano-N'-methyl-N"-(2-chloroethyl)guanidine (16.0 g, 0.1 mole) is suspended in aqueous 40 % methylamine (150 ml). O-ethyl-S-(4-methyl-imidazolyl-5-methyl)dithiocarbonate hydrobromide (29.7 g, 0.1 mole) is added thereto and the reaction mixture is heated for 30 minutes to the reflux temperature. Then the reaction mixture is slowly cooled and stirred at room temperature for 4 hours. White crystals, which separate, are sucked off and washed with water. Thus, there are obtained 17.6 g (70 %) of the desired compound, m.p. 140 to 142° C.

## Claims

1. A process for the preparation of crystalline cimetidine (N-cyano-N'-methyl-N"-{2 [(5-methyl-1 H-imidazole-4-yl)methyl-thio]ethyl)guanidine) of the formula 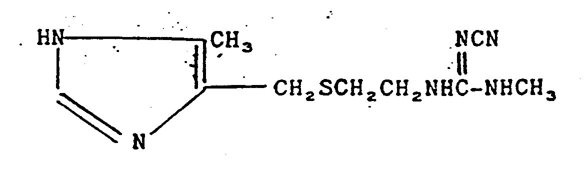 characterized in that a suspension of N-cyano-N'-methyl-N"-(2-chloroethyl)guanidine in an aqueous methylamine solution is reacted with O-ethyl-S-(4-methyl-imidazolyl-5-methyl)-dithiocarbonate hydrobromide at a temperature between room temperature and the reflux temperature of the reaction mixture, whereupon the title compound is isolated in pure form.

## Patentansprüche

1. Verfahren zur Herstellung von kristallem Cimetidin (N-Cyan-N'-methyl-N"-{2[(5-methyl-1 H-imidazol-4-yl)methyl-thio]ethyl}guanidin) der Formel 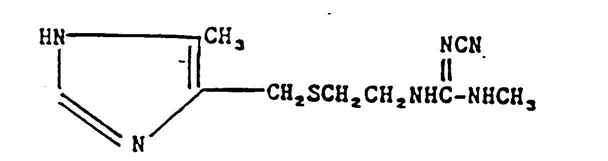 dadurch gekennzeichnet, dass die Suspension von N-Cyan-N'-methyl-N"-(2-chlorethyl)guanidin in wässriger Methylaminlösung einer Reaktion mit O-Ethyl-S-(4-methyl-imidazolyl-5-methyl)-dithiocarbo- nat-hydrobromid bei einer Temperatur, die zwischen Raum- und Rückflusstemperatur der Reaktionmischung liegt, unterworfen wird, worauf die Titelverbindung in reiner Form isoliert wird.

## Revendications

1. Procédé de préparation de cimétidine cristalline (N-cyano-N'-méthyl-N"{2[(5-méthyl-1 H-imidazole-4-yl)-méthyl-thio]ethyllguanidine) de formule 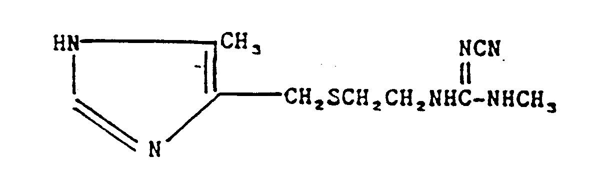
caractérisé en ce qu'on fait réagir une suspension de N-cyano-N'-méthyl -N"-(2-chloroéthyl)-guanidine dans une solution de méthylamine aqueuse avec un bromhydrate d'O-éthyl-S-(4-méthyl- imidazolyl-5-méthyl)-dithiocarbonate à une température entre la température ambiante et la température de reflux du mélange réactionnel, on isole ainsi le composé mentionné ci-dessus sous une forme pure.
